# EUROPEAN PATENT SPECIFICATION

(11) **EP 4 315 809 B1**
(45) Date of publication and mention of the grant of the patent: **02.07.2025**
(21) Application number: 22713659.5
(22) Date of filing: 14.03.2022
(51) Int. Cl.: H04L 67/12

(54) **SYSTEM AND METHOD FOR TRANSMISSION OF DATA COMPRISING A REMOTE MONITORING SERVER AND AT LEAST ONE REMOTE DEVICE**
SYSTEM UND VERFAHREN ZUR ÜBERTRAGUNG VON DATEN MIT EINEM FERNÜBERWACHUNGSSERVER UND MINDESTENS EINEM ENTFERNTEN GERÄT
SYSTÈME ET PROCÉDÉ DE TRANSMISSION DE DONNÉES COMPRENANT UN SERVEUR DE SURVEILLANCE À DISTANCE ET AU MOINS UN DISPOSITIF DISTANT

(30) Priority: 30.03.2021 US 202163167710 P; 17.05.2021 EP 21174001
(43) Date of publication of application: 07.02.2024
(73) Proprietor: BIOTRONIK SE & Co. KG, 12359 Berlin (DE)
(72) Inventor: HORTON, James, Portland, Oregon 97225 (US)
(74) Representative: Biotronik Corporate Services SE
(86) International application number: PCT/EP2022/056425
(87) International publication number: WO 2022/207284

(56) References cited:
- CN-U- 202 750 239
- US-A1- 2009 063 187
- US-A1- 2014 003 254

## Description

This invention disclosure describes a system and method for transmission of data comprising a remote monitoring server (RMS) and at least one remote device. The data comprise medical data.

Medical devices such as implants as known in the art can be coupled with various external devices to exchange data. For example, an implantable medical device can be coupled with a remote device such as a programmer that allows a health care professional (HCP), such as a clinician, to adjust the implantable medical device's settings. For coupling a magnetic switch can, for example, be used, which is placed over the implantable medical device. The magnetic switch has a permanent magnet whose magnetic field is detected by the implantable medical device and puts it into a coupling state in which the implantable medical device is coupled to the programmer.

Medical devices commonly make use of off-the-shelf connectivity components, standard communication protocols and public networks. When using an OEM device such as a smartphone, they use that device's native connectivity applications. Though these systems transmit data only, with relatively small payloads and transfer rates, they tend to use communication protocols designed for the much higher data demands of combined voice/data applications.

**In** addition, in a wireless monitoring system, a patient device is known to connect wirelessly to a medical device when located near the medical device. Such patient devices are used, for example, to download patient data at regular intervals (e.g. daily) from the implantable medical device and forward it to a service centre. At the service centre, the data are pre-processed and, if necessary, transferred to a user, e.g. an HCP.

A patient remote device may, for example, be equipped with a wake-up function to switch an associated implantable medical device from a resting state to an active state in order to establish a communication link with the implantable medical device. The patient device for example emits a wake-up signal in a radio frequency (RF) range, which is received by the implantable medical device, whereupon it is activated for data communication with the patient device.

In addition, systems exist providing the possibility for a patient to initiate a recording of data by the implantable medical device. If, for example, a pacemaker patient does not feel well, e.g., if the patient feels dizzy or experiences a tachycardia, the patient can trigger the recording of an ECG by the implantable medical device, e.g. by actuating a remote control. The data recorded in this way is transferred directly to an HCP or to a service centre the next time data is retrieved from the implantable medical device, for example during an examination at the HCP or via an automatic query of the patient remote device. Although some implantable medical devices have means for triggering an interrogation when the patient is remote from an HCP, the patient generally is involved as actor able to trigger the remote interrogation, the HCP currently having limited ability to trigger the remote interrogation by him-/herself.

In a typical workflow, as provided by some products currently available, a patient, who feels symptomatic, may wish to send a report to a clinic, or the clinic may request that the patient generate a report. In either case, the patient follows the process to initiate the patient device to trigger the interrogation. This requires either that the patient places an interrogation wand over the implanted medical device, or if available, the patient device can connect to the implanted medical device via wireless means. The implanted medical device processes the interrogation and transmits data to the patient device. The patient then triggers the patient device to transmit an interrogation message, for example, to a service centre, or if available, the patient device initiates the transmission to the service centre automatically.

In another example, an HCP wishes to have a look at actual patient data and/or data of the patient's medical device in order to prepare for a follow-up of the patient's medical device. For that, the HCP connects with a service centre and/or retrieves data of the respective patient and the patient's device using his/her remote computer.

For communication medical devices, the service centre and patient's or HCP's remote devices typically use a single communication protocol (e.g. LTE or WLAN) and are not able to change their configuration once a session is started. For example, document US 7,865,242 B2 describes to equip a patient device with an automatic routing/dialling module connected to a data communication interface and being adapted to establish an automatic access to a modem connected to the data communication interface by automatically selecting one of a plurality of possible connection parameters, the connection parameters to be selected include an individual modern, if more than one modem is connected to the data communication interface, or a prefix number for remote access to remote access device over a public network, or both. The patient device is not able to change its configuration once a communication session is started.

Other proposals to improve connectivity are related to the hardware used in each component, such as battery, antenna and telemetry chip etc. As an analogy, these proposals look "inwardly" to the components of the medical device and associated medical device infrastructure; they do not consider and attempt to control or modify the outside communication processes.

Remote data transmission systems comprising medical devices, which rely on public networks for their connectivity, are frequently subject to reduced or lost connectivity. In some cases, this will require action by the patient to restore the communication. Historically, requiring the patient to be a participant in establishing connectivity leads to a reduction in system compliance. This in turn leads to reduced HCP and patient satisfaction and acceptance.

Most modern connectivity-capable devices, e.g. smartphones, have the ability to use multiple connectivity paths (e.g. switch between WLAN and LTE or UTMS, or between available WLAN networks, or between available cellular network bands, etc.). These devices typically have native algorithms that are intended to allow them to automatically choose between available paths. However, these native algorithms have their shortcomings. They tend to be slow to respond or non-responsive to changes in connection during a session (e.g. device user moves from an area of good connectivity to poor connectivity; signal drop-out occurs on a particular network; etc.). In these circumstances, the user is frequently required to intervene in the process (e.g. to manually switch between networks, or between communication protocols, etc.).

A real world example of this is when a smartphone user, while browsing the Internet, walks into a large building. While outside the building, their smartphone is using LTE to connect to the Internet. When they enter the building, LTE connectivity will be reduced by the building structure but a strong WLAN signal from a public network is available. The smartphone algorithm automatically switches over to the strong WLAN signal. However, the building owner requires that one acknowledges use of the network before it will connect, but this acknowledgement is contained within a browser window that may not automatically open. If the user is not aware of this problem and does not provide the required acknowledgement, connectivity stops.

A similar example occurs in a location with many concurrent users (e.g. at a sports arena, conference center, etc.). In this scenario, a strong WLAN signal exists, but the bandwidth is crowded by the many concurrent users. The smartphone will default to the strong Wi-Fi signal, but the session can't progress due to poor bandwidth access. In this scenario, the problem can frequently be resolved by user intervention, namely turning off WLAN and allowing the session to occur with the cell network, instead.

CN 202 750 239 U relates to a wisdom medical wireless gateway based on an internet of things technology. The gateway comprises a microcontroller, a power supply transformation module and a wireless sensor network center node. The gateway is characterized in that the microcontroller is connected with a WIFI control transmit-receive module and a 3G communication control module through a network selection control circuit, the WIFI control transmit-receive module is connected with a 2.4G transmit-receive antenna, and the 3G communication control module is connected with a 3G transmit-receive antenna.

US 2014/0003254 A1 relates to a wireless access network selection. The selection process may include arbitrating use of a plurality of wireless networks according to their performance capabilities. The performance capabilities or parameters may be identified from information broadcasted from the wireless networks. Mobile devices capable of wirelessly connecting to the networks may identify the performance capabilities prior to connecting to the wireless networks.

Accordingly, there is a need in the state of the art to provide a system and method which improve native communication algorithms, to eliminate the need for user intervention in creating and maintaining a stable communication session and to provide a faster, more stable execution of connectivity use cases by automatically adjusting for bottlenecks.

The above object is solved by a system having the features of claim 1, a method with the features defined in claim 8, a computer program product with the features of claim 12 and a computer readable data carrier with the features of claim 13.

Particularly, the above object is solved by a system for transmission of data comprising a remote monitoring server (RMS) and at least one remote device of the group comprising at least one patient remote device (PR) and at least one health care professional (HCP) remote device (CP), wherein each of the at least one remote device and the RMS comprises a communication module that is configured to establish and maintain a communication connection between the RMS and the respective at least one remote device through one of a at least two communication paths for data transmission, wherein each communication path uses a different communication protocol and/or has a different service set identifier, wherein the communication module is configured to determine and analyze values of at least one of a plurality of connectivity parameters of each of the at least two communication paths between the respective remote device and the RMS and to automatically choose one communication path of the at least two communication paths for establishing and/or for maintaining the communication connection between the respective remote device and the RMS based on the analyzed values of the at least one connectivity parameter. The RMS and the at least one remote device are referred to as elements of the system in the following.

In one embodiment, the system comprises at least one medical device, wherein each medical device comprises a communication module in order to send data determined by the medical device to the at least one patient remote device. In one embodiment, the communication module of the medical device has a sender or a transceiver that uses a pre-defined communication path using a pre-defined communication protocol in order to uni- or bidirectionally communicate with the patient remote device. The medical device may be an implanted medical device (IMD) which may be at least partly implanted in a patient's body. Alternatively the medical device may be attached to the patient's body or a wearable device.

As indicated above the inventive system comprises for example the following components:
- A Remote Monitoring Server (or RMS), for example a "Neuro Service Center" (NSC) in one embodiment. The RMS is, for example, a hardware and/or cloud-based service centre that facilitates Remote Programming (RP) and data interrogation of the at least one medical device, and may comprise a central data repository of data collected by the at least one CP, the at least one PR and/or the at least one medical device, wherein the hardware may comprise at least one computing device or a network of a plurality computing devices, wherein the RMS further comprises the computing functionality described herein; the RMS is a functional unit that performs substantial computations, including numerous arithmetic operations and logic operations without human intervention and may comprise, such as, for example, a desktop computer, a server computer, clusters/warehouse scale computer or embedded system; and
- At least one remote device, wherein the remote device is of the group comprising at least one patient remote device (PR) and at least one HCP remote device (CP), wherein
   ∘ A Patient Remote (computing) device (PR) may serve as a transceiver to route communication between the medical device and the RMS, and provide to the patient some aspect of local control of the therapy process. For example, it gives the patient the ability to change the active therapy program of a medical device, for example a medical device for SCS, control its stimulation amplitude, turn stimulation on/off, and view battery status. It may also serve as an external programmer for the medical device;
   ∘ An HCP remote (computing) device/programmer (CP) used by HCPs or field technical representatives to communicate with the RMS in order to receive data from the RMS, or to communicate with the PR, in order to, for example, receive data from the PR or to program the medical device remotely. The communication to the PR may be provided via the RMS. The CP may be physical (i.e. dedicated computing device) or virtual (e.g. via web-based interface to the system, secure connected data reporters); and, if applicable,
- At least one individual-patient medical device, for example an IMD, also known as "therapy device".

For communication, public or private communication network(s) (PCN) is (are) used and in each CP an HCP device user interface (CUI), e.g. GUI at the physical CP or web portal at virtual CP and in each medical device a PR user interface (UI). The RMS may further manage deployed at least one PR and at least one CP, which are together called external devices (ED) in the following.

In one embodiment the PR remote device and/or the HCP remote device may be a smartphone comprising an app realizing above and below described functionality.

For data/signal processing each of the CP, RMS, PR, medical device comprises at least one processing unit which is generally regarded as a functional unit of the respective component, that interprets and executes instructions comprising an instruction control unit and an arithmetic and logic unit. The processing unit may comprise a microprocessor, a controller, a digital signal processor (DSP), an application specific integrated circuit (ASIC), a field-programmable gate array (FPGA), discrete logic circuitry or any combination thereof. Alternatively or additionally, the processing unit may be realized using integrated dedicated hardware logic circuits, in particular in the case of a medical device due to the small size and extreme power limitation.

The above system provides improved medical device remote monitoring system connectivity reliability, greater medical device remote monitoring system speed and improved medical device remote monitoring system automaticity. This leads to better user experience with a networked patient remote device and, similarly, a networked medical device, a system that is better able to autonomously react to the connectivity environment which is advantageous versus the off-the-shelf performance. Accordingly, user (patient, HCP) satisfaction and patient's compliance increases, and use-cases that demand continuous connectivity see improved performance. Further, the system has a better data throughput and saves energy because its better reliability reduces idling time.

Each component (i.e. the RMS and the at least one remote device) comprises a communication module for uni- or bidirectional communication as indicated above. The communication is mainly extracorporeally, with the medical device it may be partly intracorporeally, if the medical device is an IMD. The communication is provided by one communication technique wirelessly via the air using electromagnetic waves, for example, EDGE, EV-DO, Flash-OFDM, GPRS, HSPA, LoRaWAN, RTT, UMTS, Narrowband IoT, Bluetooth, WLAN (WiFi), ZigBee, NFC, LTE, Wireless USB, Wibree (BLE), Ethernet or WiMAX in the radio frequency region, or IrDA or free-space optical communication (FSO) in the infrared or optical frequency region. Accordingly, the respective communication protocols, often with the same name (i.e. a system of rules that allows two or more entities of a communications system to transmit information via an kind of variation of a physical quantity, defining rules, syntax, semantics and synchronization of communication and possible error recovery methods and being implemented by hardware, software or a combination of both) or cooperating protocols (protocol suites) may be used, for example TCP/IP protocol suite (e.g. IPv6, TCP, UDP, SMTP, HTTP/2) also with TLS or SSL, IPX/SPX, X.25, AX.25, ebXML, Apple Talk, Bluetooth protocols (e.g. BR/EDR), Bluetooth 4.0 (BLE), ZigBee protocol, NFC protocol, IEEE 802.11 and 802.16 protocols, etc. The communication module comprises a sender, a receiver and/or a transceiver for sending and/or receiving the respective communication signals. The communication module is electrically connected to the processing unit of the respective component or may be integrated within the processing unit. For example, the communication module may comprise at least one processing unit (e.g. one processing unit for each communication path realized) separate from the main processing unit of the respective component. The communication module may be electrically connected to a data memory as well. The communication module is configured to provide at least two communication paths, for example three or more communication paths, wherein each communication path uses a different one of the above mentioned communication techniques based on one specific communication protocol. As two different communication paths are regarded:
- communication techniques/communication protocols which work basically different such as, for example, Zigbee and Bluetooth, and
- different versions of the same communication technique/communication protocol such as, for example, Bluetooth 4.0, Bluetooth 4.1, Bluetooth 4.2, and Bluetooth 5, IEEE.11b, IEEE.11g, IEEE.11ax.

Alternatively, two different communication paths are provided by two communication paths which are based on the same or compatible communication protocol but have a different service set identifier (SSD). The service set is a group of wireless network devices which share an SSD - typically the natural language label that users see as a network name. A service set forms a logical network of nodes operating with shared link-layer networking parameters, the form on logical network segment.

Accordingly, the communication module of each component of the system comprises the respective hardware and software adapted to the communication techniques / communication protocols which are provided by the respective communication module. Further, it is necessary that the RMS and the at least one remote device comprise communication modules providing at least partly the same communication path (RMS and at least one remote device) in order to "talk" to each other, i.e. to establish and maintain a communication connection between each other in order to transmit data, for example data of a bodily parameter of the patient and/or data of the medical device implanted within or attached to the patient's body. Sometimes the communication techniques are downward compatible which means that a communication module with a higher version of a communication protocol "understands" a communication module with a lower versions of a communication protocol, i.e. a working communication connection can be established by communication modules comprising different versions of the same communication module. Such situations shall be covered by the present invention.

Further, the communication module of each element comprises a measuring unit or detector for different connectivity parameters such as, for example, at least one parameter of the group containing network speed, ping time, packet loss percentage, transfer rate, signal strength, success rate of communication for each of the implemented communication paths of the respective communication module. The communication module determines continuously or in pre-defined time intervals values of the respective connectivity parameter(s). The communication module analyzes these determined values and automatically chooses one communication path of the at least two communication paths based on this analysis because the values show that one of the at least two communication paths is more favorable than the other(s). This analysis may be provided prior the establishment of a communication connection between the RMS and one remote device or during maintenance of a communication connection. If the analysis is made during maintenance of the communication connection the analysis may be triggered, for example, if one or several connectivity parameters is/are above a pre-defined threshold value, below a pre-defined threshold value or within or outside a pre-defined value range. Alternatively or additionally the analysis may be provided in pre-defined time intervals. For example, progress of data transfer during a maintained communication connection is monitored. If the respective communication module observes during analysis of the transfer rate that it has dropped below a pre-defined rate threshold, it re-queries the possible communication paths to determine whether a faster means to complete the transfer exists.

The PR remote device and/or the HCP remote device may ping the respective other device via the RMS or the RMS during the maintenance of a communication connection at regular intervals to ensure that handshakes persist and that any delays are minimized.

In one embodiment, the communication module is configured to choose the one communication path based on the analysis of the values of the at least one connectivity parameter detected by itself only. This means that the decision making process resides in the component initiating the transmission; all data and processing capability required for the routing algorithm is stored locally to this remote device or to the RMS. In this embodiment, the remote device can operate in total isolation from the rest of the medical device system. Decision weighting is based on the local environment alone.

In an alternative embodiment, the communication module is configured to choose the one communication path based on the analysis of the values of the at least one connectivity parameter detected by itself and additionally based on the analysis of the values of the at least one connectivity parameter of the communication module of all similar or associated remote devices of the system. In this embodiment, the decision making process occurs in the component initiating the transmission; all data and processing capability necessary for the routing algorithm is stored locally to this device. However, its data is shared with similar remote devices of the rest of the system so as to optimize communications throughout the system. Decision weighting is based on the shared data pooled from all similar remote devices in the network.

In one embodiment, the communication module is configured to choose the one communication path based on actually determined values of the at least one connectivity parameter and based on the values of the at least one connectivity parameter determined in the past. Including values of the at least one connectivity parameter from the past enlarges the number of data and makes therefore the selection of one communication path more reliable. The connectivity parameters from the past are stored in a data memory of the at least one remote device and/or in a data memory of the RMS. In one embodiment, a pre-defined past time range is defined, wherein values of connectivity parameters determined within the defined time range are included in the analysis of the respective communication module, wherein the time range may be adapted to the systems' needs.

In one embodiment, the communication module is configured to store values of the at least one connectivity parameter determined during one communication session in a data memory of the respective at least one remote device and/or in a data memory of the RMS at the end of the respective communication session. For example, each time a message is successfully relayed from a remote device to the RMS (or back), the transmitting device learns from the experience. The device tracks transmission success and utilizes this historical data to weight the communication path selection towards the path most likely to provide success in any given environment (e.g. preference to attempt connection via Wi-Fi first, rather than LTE, when in an Emergency Department).

Each component, including the medical device, if applicable, may comprise the data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. The data memory of the RMS is referred to as central data repository in the following, as well.

In one embodiment, the communication module is configured to use at least one AI algorithm (AI = artificial intelligence) for analysis of the determined values of the at least one connectivity parameter. For example, in an enhancement of the processes described the communication module makes use of neural network(s) to allow it to "learn" optimal means to execute each process.

In one embodiment, the communication module is configured to store the determined values of the at least one connectivity parameter together with the location and/or time of determination by the respective at least one remote device and/or RMS, for example, a GNSS value of the location. Accordingly, the determined values of the at least one connectivity parameter are linked to the respective location information and/or time information. This allows to favor one communication path based on the actual location and/or time for the at least one remote device at least as a first approximation.

In one embodiment, the system comprises an integrated network of the at least one medical device, for example an implanted medical device (IMD), the at least one patient remote device, e.g. a transceiver and/or therapy control device, with a patient user interface (UI), the RMS forming a service centre comprising a central processing (and optionally admin) unit, and the at least one CP as an HCP device (e.g. a computer, a tablet or a notebook, also called programmer) with a HCP user interface (CUI) for remotely programming the medical device. The mentioned system components may communicate via private or public communication networks. The system may be used for remote programming of the medical device using the CP with a live connection via the RMS and the PR to the medical device.

In one embodiment, the system may be used for triggering a real-time interrogation of the patient's medical device current data, and for remote analysis of current and historical patient data, all done during a communication session between the CP and the medical device via the RMS and the PR.

The invention may be used in multiple areas of medical device application, e.g. spinal cord stimulation (SCS), cardiac rhythm management (CRM), and many other medical fields that utilize an implanted medical device e.g. deep brain stimulation (DBS), occipital nerve stimulation (ONS), trigeminal nerve stimulation (TNS), vagal nerve stimulation (VNS), phrenic nerve stimulation, gastric electrical stimulation, and sacral nerve stimulation (SNS)..

The following features are also / may also be implemented in any combination with each other:
- HCP-triggered request for the interrogation of the chosen medical device, wherein the interrogation may be initiated remotely from one or more CUI, e.g. (1+N) CUI where N is a natural number; N = 0, 1, 2, ....
- The data collected by the interrogation may be viewed remotely at (1+N) UI or CUI.
- Data derived from interrogation are a combination of subjective and objective data.
- Data derived from interrogation are a combination of current and historical data.

In one embodiment one or more (e.g. all) steps in the sequence of establishing communication connections may require a "handshake" process that may 1.) ensure cybersecurity and 2.) provide automatic data persistence, communication persistence and communication repetition to ensure that the communication between each component is completed.

The medical device may comprise at least one detector detecting at least one bodily parameter of the patient. The detector is connected to the processing unit for processing the determined bodily parameter.

Each component and the medical device may comprise further units such a power supply, e.g. a battery.

The medical device's, in particular, the implantable medical device's units may be contained within a hermetically sealed housing.

For remote programming (RP) or interrogation using the CP, in one embodiment the use of single, serial handshakes between each component of the system is significantly reduced. Each component is designed to autonomously maintain a continuous connection to its respective neighbours, via an on-going session that persists for the duration of the RP or interrogation process, wherein the medical device has bidirectional communication link to the PR, the PR has a bidirectional communication link to the RMS and the RMS a bidirectional communication link to the CP, for example
- Medical device and PR maintain, for example a Bluetooth connection while they are in proximity.
- PR and RMS maintain, for example a TCP/IP network connection over the internet while mobile networks are available.
- CP and RMS maintain, for example a TCP/IP network connection over the internet while mobile networks are available.

Further, feedback loops exist between the components to maintain a closed loop of one component with the component adjacent to it in the communication pathway, to proactively assess the status of the communication process and to automatically and immediately process information when received. Each link between one component to the next transmits new data to the next hop in chain as soon as new data is available, thereby reducing transmission latency to (nearly) zero. Additionally, in case of connectivity loss, any system component may send new buffered data, if necessary, via a newly established communication path as soon as connectivity to the neighbour is restored. The system, i.e. each of the at least one CP, the RMS and at least one PR comprises a data buffer of sufficient depth to sustain occasional loss of connectivity, and to allow the transmission of large datasets.

The RMS may be hosted by a service provider, e.g. a company external from a clinic. The RMS may comprise at least one of the following services:
- Repository of data collected by ED and medical devices;
- Repository of keys and certificates for communication of the elements of the system and the HCP or patient via UI or CUI with the system
- Facilitates remote programming by routing data to/from their intended destinations;
- Communication with an external push notification service(s)
- CP fleet management (e.g.via Airwatch and processes to manage CPs);
- Medical device instance authentication service;
- HCP, technician and patient authentication service; and
- Database analytic tools.

As indicated above, the RMS may serve as a repository of data collected by EDs and medical devices. The latter may comprise home monitoring (HM), which originates from the medical device and is routed to the RMS via the PR. In one embodiment the HM data may be encrypted by the medical device and may only be decrypted by the RMS. In this embodiment, the PR does not have any key to decrypt the data. RMS's "certificate and key management system" (CKMS) may be used to decrypt the data.

Further, after the above mentioned communication connections are established from the CP to the chosen medical device as indicated above, the RMS is used as the conduit to route RP commands from the CP to the medical device and/or return responses and status updates from the medical device to the CP.

A hardware and/or cloud server (e.g. Couchbase^{®} server) may be used to store the data in the RMS. The RMS notifies EDs to pull data from the server via push notifications. RMS sends a push notification request to an external notification service (i.e., Microsoft Azure, Google Firebase Cloud Messaging), which in turn is sent to a specific ED.

In one embodiment, a subset of data is copied from the central data repository of the RMS to a data warehouse to support internal business functions such as technical forensics, quality monitoring, and clinical studies.
- CKMS may be used to generate and store secrets for every manufactured medical device. Secrets, e.g. encryption keys and authentication credentials, are generated during manufacturing and stored into the medical device. Generated secrets are used to cipher data when:Remote programming data is sent from the RMS to the medical device;
- HM data are sent from the medical device to the RMS.
- RMS uses an authentication management and access service, e.g.Keycloak, for PR authentication. During provisioning, a PR creates a username and password on the authentication management and access service. Subsequently, PRs submit their credentials to the service for an access token, for example, based on JSON Web Token (JWT). The token is then presented to RMS Sync Gateway to gain access. This follows the OAuth 2.0 authorization code grant model. OAuth is an open standard for access delegation, for example, used as a way for Internet users to grant websites or applications access to their information on other websites but without giving them the passwords.

The RMS may provide CP user management and authentication using a Microsoft Azure Active Directory (AAD) which may be integrated in the RMS or may be realized as an external unit connected with the RMS. In the latter case, the RMS interacts with AAD for HCP and patient management operations.

In one embodiment, CPs may follow the OAuth 2.0 authorization code grant model to gain access to the RMS, where AAD acts as the authorization and token endpoints. Once authenticated, the CP is given an access token, which in turn passes to RMS Sync Gateway to gain access.

Medical devices may send/receive data to/from the RMS, via the PR, such that the PR may not decipher the data. Each medical device has unique keys assigned to it at the time of manufacturing.

EDs may communicate to the outside world via above mentioned public communication connections. This introduces a large attack surface that may impact the ED-to-medical device programmability. However, CPs may be protected by the following:
- Malware protection and firewall (e.g. Windows Defender malware and firewall).
- CP users are granted user rights and thus not able to install software.
- Internet resources are whitelisted to resources that are needed for its functionality (e.g., NSC, Microsoft, MDM provider, etc.)
- Access to other internet resources, such as Microsoft Store, is inhibited or limited to privately published apps.

PRs may be protected by the following:
- No internet browsers installed.
- Messaging and phone apps disabled.
- Access to other internet resources, such as Google Play Store, is inhibited or limited to privately published apps.

In one embodiment, Couchbase^{®} may be used as the database engine used to store and share data in the RMS. EDs may use Couchbase Mobile, and the RMS may use Couchbase^{®} Sync Gateway and Couchbase^{®} Server. This setup allows for data replication between the mobile client (EDs) and the RMS.

On the PR information may be stored, e.g. in the Couchbase^{®} instance, pertaining to the medical device, which is associated with such aspects as therapy program settings, battery state of charge (SoC), connection state, etc. This data, in turn, is replicated at the RMS.

In one embodiment data transmission between ED and RMS is encrypted, as well as communication link between the ED and medical device. For example, a 2-factor authentication is used when the CP user (e.g. the HCP) selects a chosen patient's medical device to remotely program. In one embodiment, the RMS is configured to store and/or manage the authentication and/or encryption keys and/or certificates for the at least one CP, at least one PR, at least one medical device as well as for the at least one user of the system, for example, patient, HCP and/or technician. For that, the RMS may integrally comprise hard- and/or software solutions for that or the RMS may be connected to respective hard- and/or software solutions. For example, Microsoft Azure may be responsible for managing this on the backend, and Microsoft APIs may be responsible for managing user input on the CP side (i.e., login mechanism, communication with Azure, handling of 2-factor input, etc.).

In one embodiment, once the CP user has been authenticated, an authentication service of the system returns one unique signed token, e.g. the above mentioned JWT token, to the CP (referred to as the remote programming session token). The CP provides this token to the RMS during every remote programming transaction during the remote programming session. The one unique token is valid for the duration of the current remote programming session; subsequent remote programming sessions require a new unique token, which means the HCP having to go through the authentication sequence again. Using such tokens allows decentralizing authentication and authorization.

In one embodiment, the at least one PR may be not configured to continuously poll the RMS for changes, as that would result in unnecessary overhead on both the PR and RMS (i.e., data usage from the PR's perspective and request processing from the RMS's perspective). Instead, the RMS may interface with an external push notification system, such as Google's Firebase Cloud Messaging system, to send a notification to the target PR corresponding to the chosen medical device. Conceptually, for example, prior or at the beginning of establishing a communication connection or at the beginning of the remote programming session the target PR receives a push notification from the RMS. This notification serves as a trigger to the PR to poll the RMS server at a faster rate. In one embodiment, the notification does not contain detailed information, such as any identifying or secret data.

In one embodiment, a remote programming session begins with the CP user (e.g. the HCP) logging into their CP. Regardless of a session type, all CP users must log in. After logging in, the CP user will be able to select the option to start a remote programming session, which will present them with a list of patients they may remotely program.

From a hierarchal standpoint, patients may be organized by their clinic. CP users may be associated with one or more clinics. Therefore, CP users may only remotely program patients' medical devices for clinics that they are associated with.

The following data may be transmitted during a communication session (i.e. "signals"). Please note that these data will differ dependent upon the embodiment. The following data may refer partly to a communication within an SCS system which is described here as a typical embodiment.
a) Medical device data
   - Medical device data (serial number, medical device type)
   - Therapy-related data (e.g. therapeutic data (packed parameter values, packed data elements, and interface parameters), lead impedance, offset measurements, therapy program selection, stimulation on/off, enable/disable MRI mode, battery State of Charge (it tells you whether or not you can deliver the therapy), status changes, lead info/configuration ("blob" and interface data), medical device alerts, IMD EC App Status, stimulation usage data (used for statistics)
   - Personal data (patient name, physician name, hospital, implantation date of the medical device, indication, lead position, lead type and extension information, implanted system component traceable IDs
   - Notes
   - Patient Identifier (unique ID, serial number of medical device)
b) Technical data
   - Technical data/logs (reset, battery, etc.)
   - System-based events
   - Battery history log
   - MRI mode
   - Magnet history
   - medical device reset log
   - ED application log
   - application logs
   - Error logs
   - Audit logs

The RMS is configured transmits at least part of these data from the RP while the RMS transfers data with the CP.

In one embodiment, for remote programming of the chosen medical device the CP is configured to produce a single program containing the updates and/or changes of the medical device's parameters and to transmit the single program to the medical device via the RMS and the corresponding PR. Further, in one embodiment the RMS is configured to encrypt the single program received from the CP, wherein the corresponding PR is configured to transmit the encrypted single program to the chosen medical device. Accordingly, the PR cannot decrypt the single program. Decryption is provided by the medical device.

In one embodiment, the RMS is configured such that its central data repository comprises data having different security level, wherein one example of data with a lower security level are pseudonymized data. Data with a higher security level are personal or private data such as names, addresses, user ID etc. These data need higher protection and can only be accessed by persons having a higher security level.

Further, the above object is solved by the method for transmission of data in a system comprising a remote monitoring server (RMS) and at least one remote device of the group comprising at least one patient remote device (PR) and at least one HCP remote device (CP), wherein each of the at least one remote device and the RMS comprises a communication module that establishes and maintains a communication connection between the RMS and the respective at least one remote device through one of a at least two communication paths for data transmission, wherein each communication path uses a different communication protocol and/or has a different service set identifier, wherein the communication module determines and analyzes values of at least one of a plurality of connectivity parameters of each of the at least two communication paths between the respective remote device and the RMS and automatically chooses one communication path of the at least two communication paths for establishing and/or for maintaining the communication connection between the respective remote device and the RMS based on the analyzed values of the at least one connectivity parameter, wherein the plurality of connectivity parameters comprise, for example, at least one parameter of the group containing network speed, ping time, packet loss percentage, transfer rate, signal strength and success rate of communication.

In one embodiment, the communication module chooses the one communication path based on the analysis of the values of the at least one connectivity parameter detected by itself only.

In one embodiment, the communication module chooses the one communication path based on the analysis of the values of the at least one connectivity parameter detected by itself and additionally based on the analysis of the values of the at least one connectivity parameter of the communication module of all similar remote devices of the associated system.

In one embodiment, the communication module chooses the one communication path based on actually determined values of the at least one connectivity parameter and based on the values of the at least one connectivity parameter determined in the past.

In one embodiment, the communication module stores values of the at least one connectivity parameter determined during one communication session in a data memory of the respective at least one remote device and/or and in a data memory of the RMS at the end of the respective communication session and/or
the communication module uses at least one AI algorithm for analysis of the determined values of the at least one connectivity parameter and/or
the determined values of the at least one connectivity parameter are stored by the communication module together with their location and/or time of determination by the respective at least one remote device and/or RMS, for example, the GNSS value of the location.

The above embodiments of the data transmission method have the same advantages as the above system. Embodiments of the system indicated above may be realized in the data transmission method analogously. It is referred to the above explanation of the system in this regard.

The above method is, for example, realized as a computer program which comprises instructions which, when executed, cause the processing units (processors) of the components of the system to perform the steps of the above method which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, a computer program product is disclosed comprising instructions which, when executed by the processing unit, cause the processing unit to perform the steps of the above defined method. Accordingly, a computer readable data carrier storing such computer program product is disclosed.

All algorithms above can contai at least one parameter of the following group:
∘ Use case in operation,
∘ Networks available,
∘ Current measured network speed(s),
∘ Historical measured network speed(s),
∘ Current ping response time,
∘ Current packet loss percentage,
∘ Current data transfer rate,
∘ Current signal strength,
∘ Global Navigation Satellite System (GNSS) location,
∘ Time at current location,
∘ Battery State of Charge, as compared to acceptable lower limits for the particular device (e.g. a certain minimum level of charge must be maintained in a therapy device for the performance of therapy, rather than for communication)
∘ Historical success rate of communication for each of the available communication paths of the respective communication module. This will be used as a weighting factor in choosing the communication path, i.e. communication paths with a high average level of success will be weighted higher than those with a lower average success rate.
∘ Selection as to whether the communication module shall determine continuously or in pre-defined time intervals values of the respective connectivity parameter(s). This is the means by which the system determines whether it needs to change connectivity routing.

In an embodiment where only a single device is making the selection of connectivity path, its weighting is "1".

In embodiments where multiple devices are involved in the decision process, each individual device has a weighting of <1 in the decision process, where the sum of all weightings equals 1. Individual weighting is based upon the relative "value" of that component in the overall system, as determined during system design and testing.

### Decision making systems (for AI-Algorithms):

To automatically and dynamically select and maintain optimum connectivity path for a remote monitoring system. Patient input requirement should be zero and shall be minimal.

The system is able to choose the best connectivity path at a specific time based on current measurement of the values listed above, with historical performance factored into the selection.

The system continuously monitors actual performance and can change routing based on current actual performance.

The model is designed to predict real-world system dynamics. The model can make independent predictions, under normal operating conditions of public communication networks.

The algorithm is a probabilistic model; a likely algorithm type would be Random Forest, but the best selection should be made during development of the process for a specific system.

All subprocesses involved in the end to end creation and communication of the data payload are modelled and measured during the function of the overall process.

Temporal resolution is determined via initial system development and refined over time based upon measured system performance.

The present invention will now be described in further detail with reference to the accompanying schematic drawings, wherein
- Fig. 1: shows one embodiment of the inventive system and the steps establishing the communication between the components of the system;
- Fig. 2: depicts another sketch of the inventive system;
- Fig. 3: shows a remote programming overall workflow;
- Fig. 4: shows the flow of events for transmission of data to the PR during RP;
- Fig. 5: depicts an example of 2-factor authentication using push notification based approval;
- Fig. 6: depicts a flow diagram of initiating an RP session (CP to PR);
- Fig. 7: shows a sequence diagram of initiating an RP session (CP to PR);
- Fig. 8: shows a flow diagram of RP session, namely PR to CP response;
- Fig. 9: depicts the RP session communication CP to PR to medical device;
- Fig. 10: shows a sequence diagram of RP with transmission acceptance;
- Fig. 11: shows a sequence diagram of RP with transmission rejection; and
- Fig. 12: shows a sequence diagram of RP transmission with connection loss.

In the following embodiments of the inventive system will be explained in detail. The embodiments are explained with regard to medical devices in the form of spinal cord stimulation (SCS) devices and a remote monitoring server (RMS) in the form of a Neuro Service Center (NRS) and with regard to remote programming which may include data interrogation.

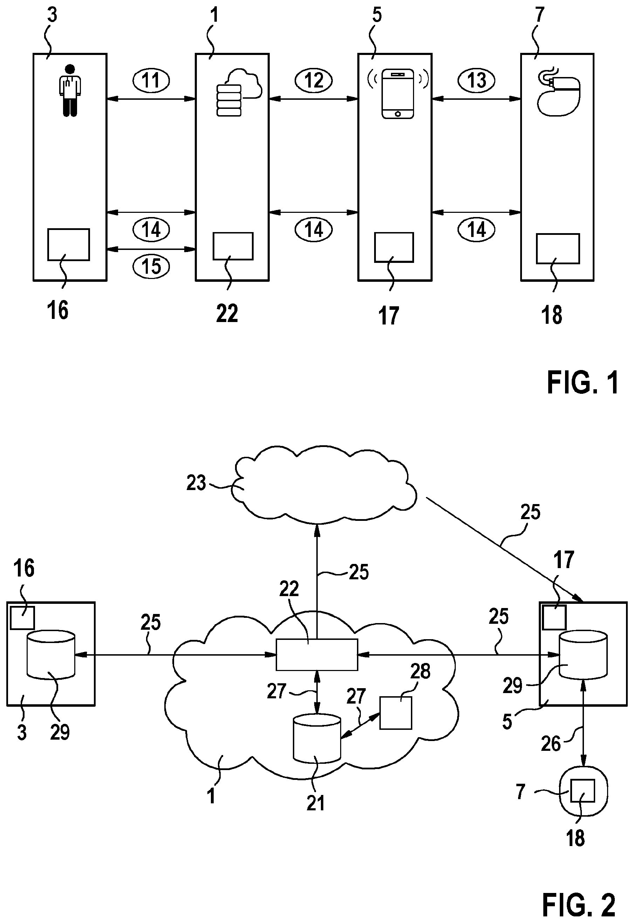

Fig. 1 shows the components of the system, namely the RMS 1, which is, for example, the NRS, at least one HCP remote device (CP) 3, at least one patient remote device (PR) 5, and at least one medical device, for example an SCS device, 7. For further information and for the properties with regard to the system components 1, 3, 5, 7 it is referred to above explanation in the general description.

The RMS 1 comprises a communication module 22, the CP 3 a communication module 16, the PR 5 a communication module 17 and the medical device 7 a communication module 18. Each communication module 16, 17, 18, 22 is configured to provide a communication connection using different communication paths, for example to communication connections via mobile wireless networks (e.g. GPRS data connection, UMTS data connection, LTE data connection), virtual private network(s) or dedicated line(s), TCP/IP via IPv4 and IPv6 (or WLAN), internet and local networks via Ethernet, electronic patient/case files (electronic medical records) via HL 7 v2 or v3 or similar. Further, suitable communication standards e.g. TLS+TCP/IP, SSL+TCP/IP or ebXML may be used. The communication module of the respective component of the system is electrically connected with its own (main) processing unit and data memory, wherein the data memory of RMS 1 is denoted as central data repository 21. Further properties of each communication module 16, 17,18, 22 is explained in the general part of the description above.

An overview of a remote programming (RP) method in which communication connections provided by the communication modules 16, 17, 18, 22 are used may be derived from Fig. 1. The remote programming method comprises the following steps. At first, the HCP triggers the communication session at his/her UI (CUI) at the CP 3 by a session request 11 which is sent to the RMS 1. Then, the RMS initiates a bidirectional communication connection 12 with the PR 5 based on a handshake with the PR 5. In the next step, the PR 5 initiates a communication connection 13 with the medical device 7 based on a handshake process, as well. Then, the medical device 7 sends a response to the CP 3 via the PR 5 to RMS 1 to CP 3 communication chain which is denoted in Fig. 1 with reference number 14. Now a continuous bidirectional communication connection is established between the CP 3 and the medical device 7 with all components now connected in real-time. Upon completion of the use case, the session initiator (HCP) indicates at CP 3 that the session is complete and signals the other components to release their handshakes (step 15). Throughout this process, only one handshake is required between adjacent components, each communication channel stays open and steady until a closing message is sent which closes all connections between the components.

In one embodiment during establishment of the communication connection the respective communication module 16, 17, 18, 22 determines values of communication parameters (see examples above) and analyses them. The respective communication units 16, 17, 18, 22 arrive at the result to use the following communication protocols / communication paths:
- Medical device 7 to PR 5: Bluetooth Low Energy (BLE)
- PR 5 to public communication network(s) (PCN), e.g. 4G LTE, WiFi 802.11
- PR 5 to RMS 1: TCP/IP
- RMS 1 to public network(s): VPN
- CP 3 to RMS 1: WLAN

If, for example there are different WLAN networks available in the area of CP 3, the communication module 16 at CP 3 uses the strongest WLAN-connection. If, during the maintenance of the communication session the actually used WLAN connection drops out, the communication module 16 automatically switches to the next-strongest WLAN-connection available.

Fig. 2 contains a different visualization of the whole inventive system. The RMS 1 comprises a Couchbase server 21 as central data repository to store data. Additionally, RMS 1 comprises a Couchbase Sync Gateway as the communication module 22 for synchronization and communication with CP 3 and PR 5. The system further comprises External Push Notification Services 23 (e.g. Microsoft Azure, Google Firebase Cloud Messaging) in order to notify the CP 3 or the PR 5 to pull data from the RMS 1 via push notifications. For that, the RMS 1 sends a push notification request to the external push notification services 23. In Fig. 2 the arrows 25 represent communication connections as indicated above, the arrow 26 a BLE connection and the arrows 27 local network connections (e.g. LAN connections). The RMS 1 further comprises a certificate and key management system (CKMS) 28 which is used to decrypt the data received from the at least one medical device 7. In one embodiment the External Push Notification Services 23 may be integrated in the RMS 1. Further, in order to communicate with the Couchbase server 21 of the RMS 1 the CP 3 and the PR 5 each comprises a Couchbase mobile application 29.

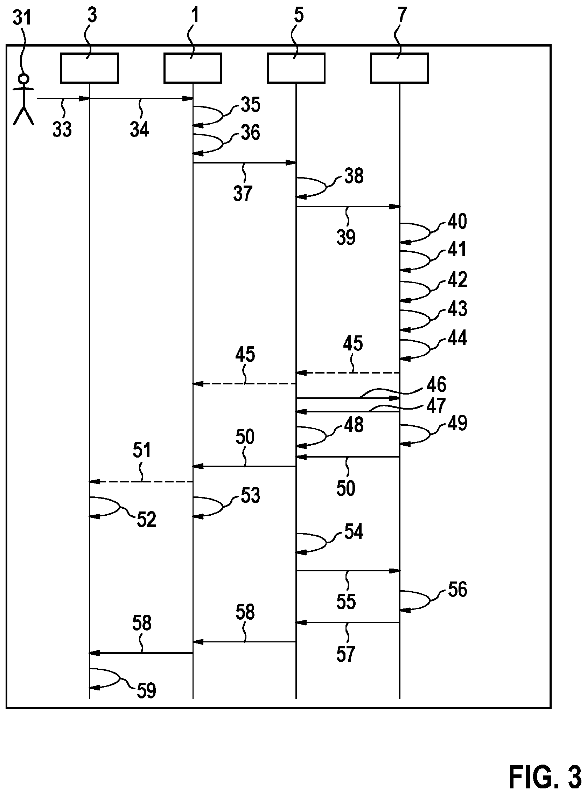

Fig. 3 shows an overall workflow for remote programming which is explained in further detail in the following. The workflow for remote programming works provided the continuous communication connection is previously established between the CP 3 and the medical device 7 after selection of a specific patient/medical device 7 for remote programming. During the continuous communication connection it may be possible, that the network drops out or that the connectivity parameters determined by the involved communication modules move into a region in which a continuous connection is not guaranteed according to historical data. Then, the respective communication module automatically switches in a well-ordered manner to another communication path, if available, so that continuous communication is provided. "Continuous communication" with regard to the embodiment presented means that the communication connection is upheld but the communication path used may be switched. The sequence of events starts with the CP user (e.g. an HCP) who commands the transmission of a therapy program using remote programming. As indicated below, the therapy program contains all changes/updates of the parameter of the medical device 7 which are to be programmed. The method describes the process uplink and downlink.

At first, after authentication of the HCP 31 at his/her CP 3 the HCP 31 configures a new therapy program for the patient to be used at its medical device 7. Then, after pressing the transmit button (step 33) the therapy program is transmitted to the RMS 1 (step 34). At the RMS the access token is verified (step 35) and a remote package containing the information of the therapy program is ciphered (step 36). This remote package is then transmitted (automatically, i.e. without further HCP or patient interference) to the PR 5 (step 37). In the next step, the patient is prompted about the fact that there is a therapy program arrived at his/her PR 5 (step 38). In the next step 39 the patient accepts the therapy program and the ciphered remote package is automatically sent to the medical device 7. The remote package is not deciphered at the PR 5 but at the medical device in step 40. In step 41 the therapy program is saved to a buffer of the medical device 7. Step 42 comprises ruling a check to the therapy program in medical device 7 and step 43 and installation and activation of the therapy program. Additionally, in step of 44 the medical device 7 generates and ciphers a remote acknowledgment signal which is to be sent to the RMS 1.

Then, using the continuous communication connection to the RMS 1, the encrypted acknowledgment signal is returned to the RMS 1 via the PR 5 (see steps 45).

Additionally, the PR 5 may automatically interrogate the medical device 7 for new data. This is facilitated in step 46, wherein this step contains an interrogation request to the medical device 7. This interrogation request may be sent via the continuous communication connection between the PR 5 and the medical device 7. In response to this request in step 47 the medical device 7 provides the PR 5 with its interrogation data. Then, in step 48 the PR 5 updates its graphical UI. At the same time the medical device 7 generates and ciphers HM PGMR frame in step 49. The PGMR frame contains detailed parameter values programmed in the medical device. In step 50 the PGMR frame is returned to the PR 5 and routed to the RMS 1. Here, the authenticity of the programmed parameter values can be checked. In the next step 51 the latest therapy entities may be pulled to the CP 3. Then, in step 52 the CUI of the CP 3 is updated and at the same time in step 53 the PGMR frame deciphered, data assembled, PPVs unpacked and PDEs as well as appropriate entities at the RMS 1 updated. This allows to check the success of the remote programming procedure on the CP and to give feedback to the HCP via the CUI.

In the next step 54 further adjustment of the medical device 7 may be provided by the PR 5, for example, in the case of an SCS a global amplitude may be adjusted. A respective signal, for example, to adjust the medical device's program stimulation amplitudes and to save the global amplitude may be sent to the medical device 7 in step 55. The respective action is provided by the medical device in step 56. In step 57 a response is sent from the medical device 7 to the PR 5 returning stimulation amplitudes. The following steps 58 returns the latest status of the PR 5 to the RMS 1 and the CP 3. Finally, in step 59 the CUI the of the CP 3 is updated accordingly.

In the process described with regards to Fig. 3 the steps 34, 37, 51 and 58 may be Couchbase, commands and responses. Further, the steps 39, 45, 50 may be provided as end-to-end encrypted commands and responses.

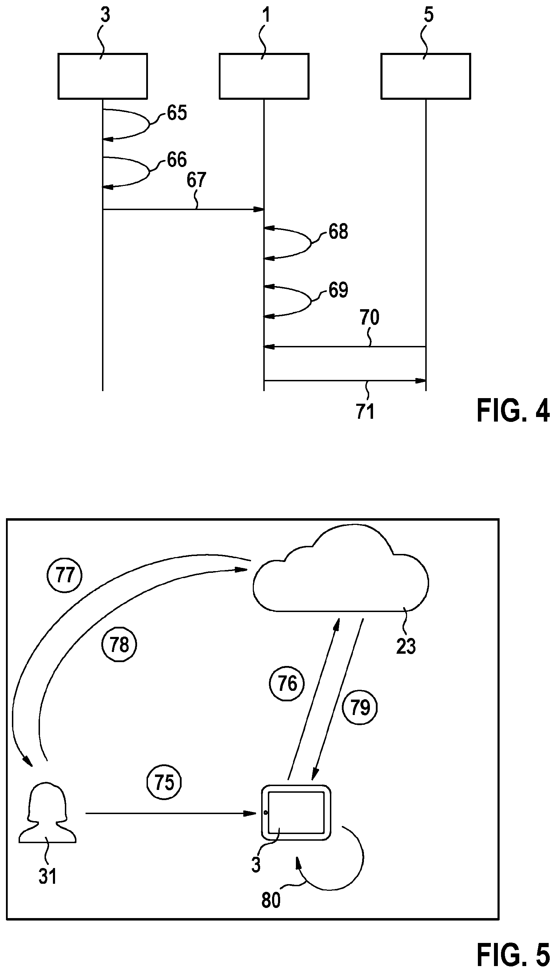

Fig. 4 describes the first steps of remote programming using the Couchbase server 21 and Couchbase mobile 29 of the CP 3 in more detail. When CP 3 commands a change to a target medical device 7 (step 65), the changes/updates of the parameters of the medical device 7 are packaged in step 66. Then, they are placed into the local a Couchbase instance by the Couchbase mobile 29 and pushed to the RMS Couchbase sync Gateway 22 and the Couchbase server 21 (step 67). Then, the package or its information is encrypted using the shared keys of the target medical device 7 provided by CKMS 28 (step 68). Because the medical device's shared secret key is only known by CKMS 28 and the medical device 7, the PR 5 is not able to decipher the package's contents. Then, the encrypted remote package is placed into the Couchbase application (step 69). The Couchbase changes are pulled and sent at the next polling interval after it has finished synchronizing with the RMS's Couchbase server 21 (see steps 70 and 71 in Fig. 4).

Prior to above programming steps the remote programming process needs to be initiated which is explained in the following with regard to Fig. 5 showing the start of a remote programming (RP) session after the HCP was logging into his/her CP 3.

Patients may be organized by their clinic. HCP, i.e. CP users, may be associated with one or more clinics. Accordingly, it may be defined that a CP user is allowed to remotely program patients medical devices only for such clinics to which they are associated. In one embodiment, a 2-factor authentication is required to be provided if the HCP selects a patient for remote programming. As indicated above, Microsoft Azure may be responsible for managing this on the back end and Microsoft APIs may be responsible for managing user input on the CP side (login mechanism, communication with Azure, handling of second factor input etc.).

The RP session starts with the HCP requesting to start remote programming in step 75 in Fig. 5. Then, in step 76 user credentials are passed for a remote programming token to the external push notification service 23. In step 77 this service sends an authorization request to the HCP's mobile device and in step 78 the HCP 31 accepts the request. Then the external push notification service 23 returns a signed access token to the CP 3 in step 79. The process then continues with remote programming (step 80).

The token (MFA token) provided by the external push notification service 23 to the CP 3 is a signed JWT token which is also referred to as the RP session token or access token. The CP 3 provides this token to the RMS 1 in every RP transaction during the RP session. The token is valid for the duration of the current RP session, subsequent RP sessions require a new token which means having to go through the authentication sequence described above again.

In the following steps, the components of the system execute connectivity steps. For that, the following messages/information are utilized by the system and/or the method:
- Follow-up ID (a unique identifier assigned to each session, for process function and tracking)
- The above mentioned multifactor authentication token (MFA, i.e. the token of the RP session determined by 2 - factor authentication).
- RpStartSession message: an entity requesting the start of a remote programming sequence and contains information to specify the target patient. This is used when the HCP selects a patient on the CUI for the remote session. RMS's remote-programming service triggers the associated PR 5 with a push notification as explained above. The PR 5 may start a replication, in case there are a lot of un-replicated data to catch up with.
- RpCpRequest message: containing the therapy program parameters
- RpPrRequest message: containing encrypted programming commands
- RpRmsStatus updates CP 3 with programming progress; captures the status of RMS's processing of the RP request with various timestamps, etc. This message contains, for example, time of RpCpRequest received, time of RpCpRequest signed by CKMS, time of RpPrRequest generated, time of push notification to PR sent, other state and error info
- RpCpStatus. The RpRmsStatus and RpPrStatus entities are used to communicate status at each hop. Each entity may contain tasks that each hop is responsible for performing, errors/status values, completion of tasks denoted by a timestamp.

The remote programming method further makes use of a "Time to Live" calculation (TTL) in its control process. As indicated above, TTL is a value in an Internet Protocol (IP) packet that tells a network router whether or not the packet has been in the network too long and should be discarded. This TTL is an absolute time in UTC. TTL is based on PR's 5 time to account for time synch mismatches between CP 3 and PR 5. TTL is calculated using current PR time acquired at session start and elapsed time since RMS data base synchronization and timeout interval. When PR receives RpPrRequest entity, it takes the TTL value and starts a timer. Expiration of TTL is communicated by setting the respective value in the RpPrStatus entity.

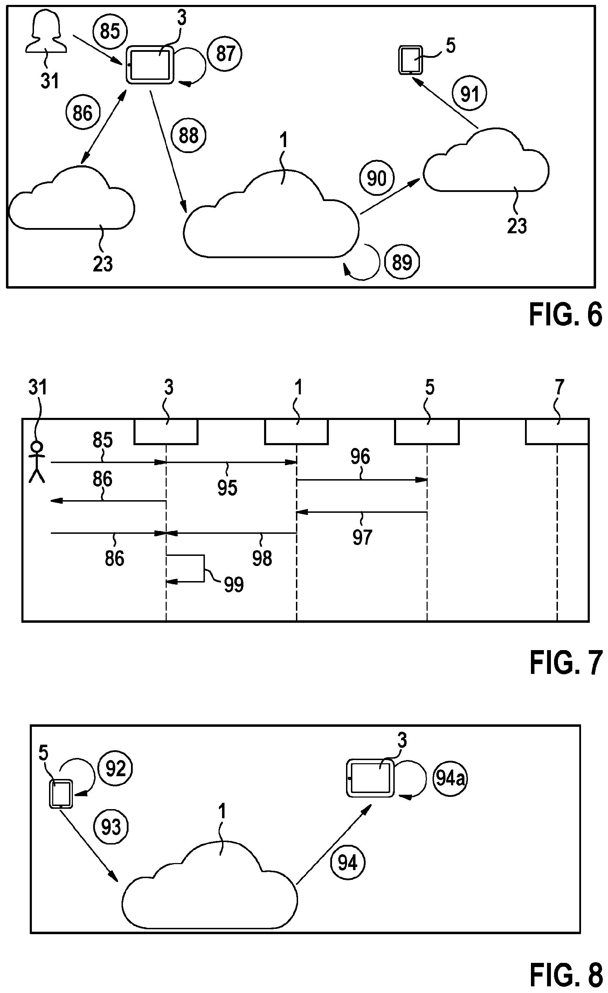

With regard to Fig. 6 further steps during initiation of an RP session are explained. This flow diagram shows that the PRs 5 are not configured to continuously poll the RMS 1 for changes as that would result in unnecessary overhead on both the PR 5 and the RMS 1. Instead, the RMS interfaces with the external push notification service 23 to send a notification to the target PR 5. For example, at the beginning of a remote programming session the target PR 5 receives a push notification. This notification serves as a trigger to the PR 5 to poll the RMS 1 (i.e. its Couchbase server 21) at a faster rate. The notification does not contain detailed information such as any identifying or secret data.

For example, the following steps may be executed for remote programming (i.e., remote follow-up). At first, the HCP selects a patient to program in step 85. The HCP navigates to the remote patient management page on the CUI found on the CP 3 and selects a patient from a filterable list of opted-in patients who the HCP is authorized to follow-up with. Then, the 2-factor authentication is provided as indicated with regard to Fig. 5 (step 86). At the same time, the CP 3 creates an entity requesting the start of an RP session (step 87), i.e. the CP 3 sends a RpStartSession command to the RMS. This means that an uplink is created from the CP 3 to PR 5. The CP 3 then waits for successful authentication, and for the PR 5 to respond with an object of the medical device 7 of the patient via RMS 1. Then, the RpStartSession is synchronized with the RMS 1 (step 88). After that, the RMS 1 identifies the target PR 5 which corresponds to the selected patient (step 89), e.g. using Google firebase. Then, a push notification request is sent to the external push notification service 23 (step 90), which contacts the PR 5 corresponding to the selected patient and the PR 5 receives the push notification and is thereby triggered to replicate with the RMS 1 at a faster rate (step 91).

As a response, which is depicted in Fig. 8, the PR 5 synchronizes with the RMS 1 (step 93) after the current PR time and medical device connection state was placed into a response entity (step 92). For example, the current battery state, therapy data such as stimulation state, and/or MRI mode state, and any medical device errors or other data of the PR database will be sent from the PR 5 to the RMS 1. Similarly, the CP 3 synchronizes with the RMS 1 (step 94), wherein the above-mentioned data are further transmitted to the CP 3. Additionally, the CP 3 quotes data from relevant entities to start the remote programming session (step 94 a). Updates to this information will be delivered on a change in any of these items. If the CP user (e.g. HCP) has not begun editing a program, the PR will also update the CP via RMS 1 with any changes to the active program, program strength, or program start strength. Relevant status information will be logged using the normal logging mechanisms. If the response of the PR 5 is received within a pre-defined time (e.g. 30 seconds) of the CP 3 sending the RpStartSession command, and the response indicates that PR is connected to the medical device and has the expected follow-up ID, the CP enters the standard programming page in remote programming mode. Otherwise, CP displays an appropriate message and stays in the remote patient management page.

Fig. 7 shows the start of the remote programming session as a sequence diagram which is explained in the following. As indicated above the HCP 31 first selects a patient who needs to receive a program update of his / her medical device 7 (step 85). The 2 - factor identification illustrated above is provided by the steps 86. Then, a start session command sent from the CP 3 to the RMS 1 (step 95). In the next step 96 the RMS 1 sends a start session notification to the PR 5. Triggered by this command the PR 5 responds by sending information received from the medical device 7 previously (step 97). This information, for example, may comprise current battery state, stimulation state, MRI mode state, or any medical device errors. This information is further transmitted to the CP 3 in step 98. Further, CUI is updated in step 99 afterwards.

In the remote programming mode CP application stages parameter changes provided by the HCP 31 to a single therapy program using the programming CUI. Remote programming differs from face-to-face in that instead of transmitting changes immediately pending changes will be highlighted to indicate to the HCP 31 the changes that will be made. Attempting to switch programs prompts the HCP as the CP user to discard his/her changes or stay on the current program. If the HCP has made all desired changes and taps the transmit button a 'transmission in progress' overlay may be displayed to prevent navigation or further program edits. This overlay may persist until programming completes will be HCP chooses to end the session.

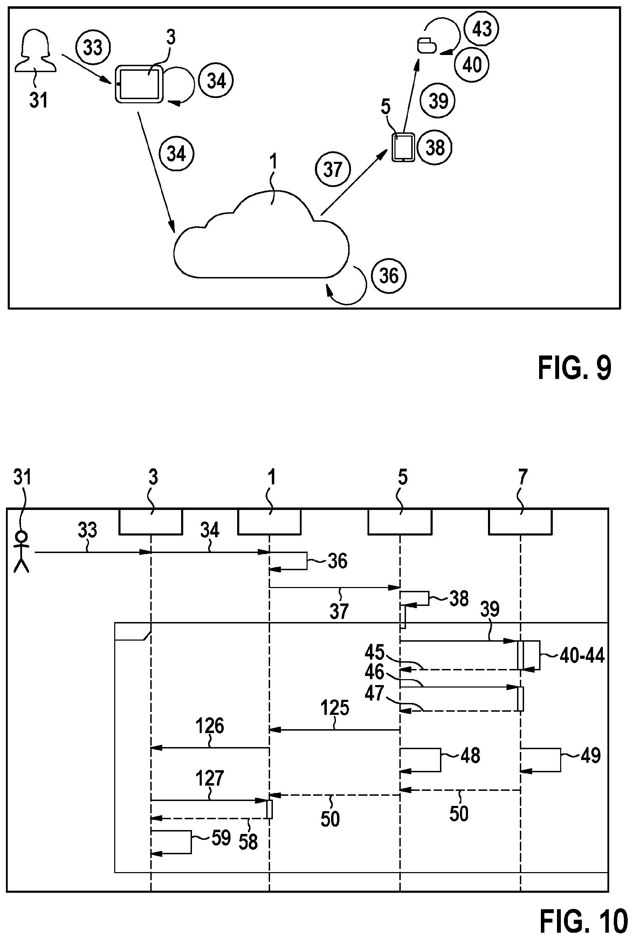

The following steps of remote programming are visualized in Fig. 9 and 10. Pressing the "Transmit" button starts the remote programming using the established communication chain CP-to-RMS-to-PR-to-medical device. The following steps describe uplink and downlink.

For the safety and comfort of the patient, all programs updated through remote programming will have their program strength set to a pre-defined minimum value. After successful programming, the patient may be required to adjust the medical device parameters, for example the strength and default strength to an appropriate level, using the standard PR 3 UI. If the remote programming session is still in progress, these changes will be reflected in the CP 3, as described above.

After pressing the transmit button (step 33, see Fig. 10), to perform the transmission, the CP 3 creates and sends an RpCpRequest entity, containing the therapy program parameters, to the RMS 1 (step 34). After program encryption (step 36), the RMS 1 then sends an RpPrRequest to the PR 5 containing encrypted programming commands (step 37) and creates an RpRmsStatus to update CP 3 with programming progress. The RMS 1 starts a Push Notification to the PR 5 so that the PR 5 may start a replication to get the RpPrRequest update as indicated above. The PR 5 does not parse this data. Upon receiving this request, if the TTL has not expired, the PR 5 asks the patient to accept or decline the program update (step 38).

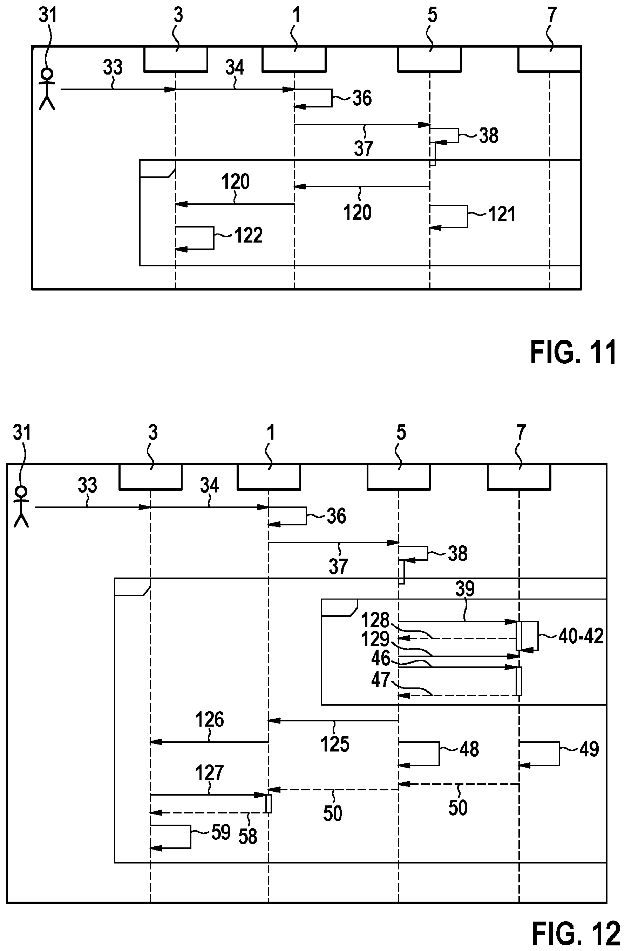

If the user rejects the update, which is shown in Fig. 11, the PR 3 will insert an RpPrStatus entity indicating the user rejected the update and a message is displayed to the PR 5 and CP 3 users via RMS 1 (steps 120). Accordingly, the UI of PR 3 and the CUI of CP 3 are updated (steps 121 and 122).

If the patient accepts the request in step 38 (see Fig. 10), the PR 3 sends the data to the medical device 7 using, for example, Bluetooth (step 39). The further steps 40 to 44 have already been explained with regards to Fig. 3. Upon completion the PR 5 will resynchronize with the medical device 7 (steps 46 and 47) and sends an RpPrStatus to the RMS 1 to indicate the result of the programming operation (step 125). If the follow-up ID or revision has changed, programming is considered to be successful, otherwise programming is determined to have failed. On successful programming, the CP 3 then receives an updated Follow-up entity from the RMS 1 which is loaded to continue the session (step 126). The following steps 58 and 59 have already been explained in connection with Fig. 3. If programming does not succeed, the CP 3 continues the session as it was prior to the "Transmit" button being pressed. The HCP uses the side menu end session option to end the session once all desired edits have been transmitted.

During remote programming, it is possible for the PR 3 to lose connection to the medical device 7 as indicated above which is depicted in Fig. 12 in step 128. In this case the communication module 17 of PR 3 tries to re-establish the communication connection using the same communication path or another communication path dependent on the analysis of the presently and previously determined values of the connectivity parameters. If the communication connection has been re-established (see step 129 in Fig. 12) and then resynchronization with the medical device 7 is provided. If after the completed resynchronization the follow-up ID or revision has changed, this will indicate successful programming. If the follow-up ID or revision has not changed the PR will retry the programming operation.

If, during remote programming, the CP 3 receives a RP 5 or medical device 7 update, or other entity, which indicates the follow-up ID or revision has changed to an unexpected value, the CP 3 will immediately notify the HCP and end the session. This scenario could occur if multiple CP's attempt to conduct remote programming sessions with the same PR 5 at the same time.

Some information needed for remote programming changes very frequently and thus the system does not/may not rely on the standard home monitoring process to transfer this information. For the purposes of RP 5, it needs to be up-to-date at the beginning of the remote programming session but it also needs to be continually updated after the remote programming session so that the CP 3 may see the patient amplitudes change in real time.

The system components are specified (e.g. memory, processing capability, transmission speed, etc.) such that they are capable of performing within the boundary requirements of the connectivity use case of the particular embodiment. For example, in the RP embodiment, the components must deliver at least the following system throughput requirements for input.

| Inputs | | | |
|---|---|---|---|
| LTE (from PR's perspective) | Download | 22.69 | Mbps |
| PR's speeds based on Speedtest's United States "2017 Mobile Report" (based on Q1-Q2 2017 data) http://www.speedtest.net/reports/united-states/#mobile | | 2836.25 | KBps |
| | Upload | 8.51 | Mbps |
| | | 1063.75 | KBps |
| BLE (from medical device's perspective) | Download | 344 | Kbps |
| The data rate is based on the assumption of 20 ms Connection Interval CI, the data payload of 215 bytes (MTU size of 251 bytes - 36 bytes of App Protocol Overhead), and 4 data packets per CI (in the case of Android). r=(I_MTU bytes * n_packets) / t_CI s | | 43 | KBps |
| | Upload | 344 | Kbps |
| | | 43 | KBps |
| RP Msg Processing Time | CP | 1 | s |
| | PR | 1 | s |
| | RMS | 15 | s |
| | External Push notification System | 2 | s |
| | TD* | 1 | s |
| medical device BLE Message Processing Latency | | 1 | s |
| RP CMD Payload Size Assuming this is the size of a single program (blob + implant data) | | 3 | KB |
| RP CMD Document Size (Payload + 50% Overhead) | | 4.5 | KB |
| medical device Interrogation Data Size Assuming PR interrogates all 12 programs after it receives an RP Success msg. | | 36 | KB |
| RS RESP Document Size (payload + 50% Overhead) | | 54 | KB |
| *- Time it takes for TD to decrypt msg (in Mesquite), rule check program, install program, activate program, and send alert to PR. | | | |

| Hop to Hop Time | | |
|---|---|---|
| RP CMD from CP to RMS | 1.00 | s |
| CP time to encrypt data + time to push data, for example, via Couchbase (ignores Couchbase comm layer activity and takes simple approach of datarate of data size). | | |
| RP CMD RMS Processing | 15.00 | s |
| Couchbase (for example) process duration + decrypting data using CP key + encrypting data using medical device key + duration to send a push notification via external service | | |
| RP CMD from RMS to PR | 3.00 | s |
| The time it takes for PR to receive push notification + time for PR to pull data from RMS (ignores Couchbase comm layer activity and takes the simple approach of transferring entire data packet based on transmission rate) + PR processing | | |
| RP CMD from PR to medical device | 1.07 | s |
| BLE transmission time + medical device processing time (receive msg decrypt msg, run rule check, install program) | | |
| PR Interrogate After RP Success Resp | 1.84 | s |
| Assuming PR reinterrogates all programs | | |
| RP RESP from PR to RMS | 1.05 | s |
| PR processing time + time to push data to RMS (assuming response sent back to NSC contains latest therapeutic data returned from medical device; ignores, e.g. Couchbase comm layer activity and takes the simple approach of transferring entire data packet based on transmission rate) | | |
| RP RESP RMS Processing | 15.00 | s |
| Couchbase (e.g.) process duration + duration to send a push notification via external service | | |
| RP RESP from RMS to CP | 3.02 | s |
| The time it takes for CP to receive push notification + time for CP to pull data from RMS (ignores Couchbase comm layer activity and takes the simple approach of transferring entire data packet based on transmission rate) + CP processing | | |

| Total Times | | |
|---|---|---|
| RP CMD from CP to PR | 19.01 | s |
| RP CMD PR to medical device + RP Install in TD (after patient ack) | 1.07 | s |
| PR Interrogation of medical device + RP RESP PR to RMS + RP RESP RMS to CP (after PR receives RP Success Resp) | 20.91 | s |
| Round Trip Time (ignoring patient ack time) | 40.98 | s |

The system further allows combining subjective (e.g. patient diary, patient surveys, HCP notes) and objective data (e.g. current patient status, patient trends and outcomes analysis, longitudinal pain data, activity-based tracking, medication use checking, statistics) into one viewable RMS database via multiple data reporters, for example, CP 3, PR 5, RMS 1 (for example NSC), Electronic Health Record system (EHR). Further, the RMS 1 provides a central service unit that serves as a central data repository of all of clinic's patients, as well as manages access rights and controls access to therapy device data according to user's log-in. The system may further comprise a web-based presentation layer, accessible from (1+N) different UI. The system may further provide HCP-accessible reporting which includes controlled access to database, configurable access rights within the database (i.e. to specific patient or groups of patients), accessible via multiple portals (CP, NSC UI, EHR UI, other). The HCP may view both current patient status as a real-time "snapshot" as well as via long-term data trends. The reporting may further comprise configurable report formats to allow customization to clinic or individual user's needs. The system further comprises a searchable patient and medical device database.

The RMS 1 may also have a user interface which may realize a closed loop, real-time communication with the at least one medical device 7. For example, if the HCP wants to know the patient's data in preparation for follow-up consult or remote assistance call with the patient the HCP triggers a respective request (update data request) from the RMS 1. This request is then transmitted to the PR 5 which in turn relays the request to the medical device 7. The medical device queries current patient and device status and transmits the report to the RMS 1 via the PR 5. The report is received and processed (i.e. the message is converted to form usable by the RMS 1, scanned for alert conditions using internal algorithms and assigned to the correct clinic and patient) by the RMS 1. Then, the HCP this able to review the new report in advance of the in-person meeting. The interface may be used to display data received via regular routine to connect data automatically (daily, monthly,...) or on purpose (e.g. preparation for in-office visit, patient adjustment, ...). Further, the RMS 1 is configured to provide a current patient status 'snapshot' collected in near real-time showing all quantitative and subjective data store for the patient. In addition, as indicated above, the RMS 1 provides online access to the PR and the medical device to collect real time data. The RMS 1 is further configured to provide the status of the patient's system and statistics collected over time (e.g. for an SCS device implanted lead integrity, present stimulation settings, the diagnostic trends related to pain or system use). In one embodiment, the user may have the ability to configure the display of the combined status such that different data trends are visible or invisible according to the user's preferences. The HCP's web interface of the RMS 1 may further provide an image of the patient, videos or audio files. Further, the interface may allow to query the database, for example for all patients or medical devices under control of the respective clinic.

In the above situation, the HCP may be, in one embodiment, in the process of pulling up a patient record of RMS 1 using their CP 3 while routing to see the patient in the Emergency Department. The session starts in the hospital lobby where good LTE connection is available, so the communication module 16 of CP 3 defaults to the LTE protocol based on the analysis of the present values of the connectivity parameters. As they enter the Emergency Department, which has poor LTE but good WLAN with automatic connectivity enabled, the communication module 16 of CP 3 automatically switches the session to the WLAN network.

In another embodiment, the PR 5 user (e.g. the patient) is reviewing content from the RMS 1 when the communication protocol handshake is lost. The communication module 17 of PR 5 pings the original network and reinstates the communication session, with minimal latency and no loss of information packets (i.e. session temporarily slows down, but session automatically picks up where it left off upon reinstituting session).

In another embodiment, PR 5 user is communicating with their HCP via their PR 5 prior to an appointment, downloading a recent survey response needed at their appointment, using LTE protocol. Once they walk into the building, the communication module 17 of PR 5 sees a relatively stronger, public WLAN signal that would normally trigger the PR 5 to switch over to WLAN. However, as this network requires a sign-on, the session is continued via LTE connectivity while at the same time the algorithm triggers a pop up informing the patient that there is a stronger WLAN network that is available if they sign on, and allows them the choice to switch.

## Claims

1. A system for transmission of data comprising a remote monitoring server, RMS, (1) and at least one remote device of the group comprising at least one patient remote device, PR, (5) and at least one health care professional, HCP, remote device, CP, (3) wherein each of the at least one remote device and the RMS comprises a communication module (16, 17, 22) that is configured to establish and maintain a communication connection between the RMS and the respective at least one remote device through one of at least two communication paths for data transmission, wherein each communication path uses a different communication protocol and/or has a different service set identifier, wherein the communication module is configured to determine and analyze values of at least one of a plurality of connectivity parameters of each of the at least two communication paths between the respective remote device and the RMS and to automatically choose one communication path of the at least two communication paths for establishing and/or for maintaining the communication connection between the respective remote device and the RMS based on the analyzed values of the at least one connectivity parameter, wherein the communication module is configured to choose the one communication path based on actually determined values of the at least one connectivity parameter and based on the values of the at least one connectivity parameter determined in the past.

2. The system of claim 1, wherein the plurality of connectivity parameters comprise at least one parameter of the group containing network speed, ping time, packet loss percentage, transfer rate, signal strength, success rate of communication.

3. The system of any of the previous claims, wherein the communication module is configured to choose the one communication path based on the analysis of the values of the at least one connectivity parameter detected by itself only.

4. The system of any of the claims 1 to 2, wherein the communication module is configured to choose the one communication path based on the analysis of the values of the at least one connectivity parameter detected by itself and additionally based on the analysis of the values of the at least one connectivity parameter of the communication module of all similar remote devices of the system.

5. The system of any of the previous claims, wherein the communication module is configured to store values of the at least one connectivity parameter determined during one communication session in a data memory of the respective at least one remote device and/or in a data memory of the RMS (1) at the end of the respective communication session.

6. The system of any of the previous claims, wherein the communication module is configured to use at least one AI algorithm for analysis of the determined values of the at least one connectivity parameter.

7. The system of any of the previous claims, wherein the communication module is configured to store the determined values of the at least one connectivity parameter together with the location and/or time of determination by the respective at least one remote device and/or RMS (1), for example, a GNSS value of the location.

8. A method for transmission of data in a system comprising a remote monitoring server, RMS, (1) and at least one remote device of the group comprising at least one patient remote device, PR, (5) and at least one health care professional, HCP, remote device, CP, (3), wherein each of the at least one remote device and the RMS comprises a communication module (16, 17, 22) that establishes and maintains a communication connection between the RMS and the respective at least one remote device through one of at least two communication paths for data transmission, wherein each communication path uses a different communication protocol and/or has a different service set identifier, wherein the communication module determines and analyzes values of at least one of a plurality of connectivity parameters of each of the at least two communication paths between the respective remote device and the RMS and automatically chooses one communication path of the at least two communication paths for establishing and/or for maintaining the communication connection between the respective remote device and the RMS based on the analyzed values of the at least one connectivity parameter, wherein the plurality of connectivity parameters comprise, for example, at least one parameter of the group containing network speed, ping time, packet loss percentage, transfer rate, signal strength and success rate of communication, wherein the communication module chooses the one communication path based on actually determined values of the at least one connectivity parameter and based on the values of the at least one connectivity parameter determined in the past.

9. The method of claim 8, wherein the communication module chooses the one communication path based on the analysis of the values of the at least one connectivity parameter detected by itself only.

10. The method of claim 8, wherein the communication module chooses the one communication path based on the analysis of the values of the at least one connectivity parameter detected by itself and additionally based on the analysis of the values of the at least one connectivity parameter of the communication module of all similar remote devices of the system.

11. The method of any of the claims 8 to 10, wherein the communication module stores values of the at least one connectivity parameter determined during one communication session in a data memory of the respective at least one remote device and/or and in a data memory of the RMS (1) at the end of the respective communication session and/or
wherein the communication module uses at least one AI algorithm for analysis of the determined values of the at least one connectivity parameter and/or
wherein the determined values of the at least one connectivity parameter are stored by the communication module together with their location and/or time of determination by the respective at least one remote device and/or RMS, for example, the GNSS value of the location.

12. A computer program product comprising instructions which, when executed by a processing unit, cause the processing unit to perform the steps of the method according to any of the claims 8 to 11.

13. Computer readable data carrier storing a computer program product according to claim 12.

## Patentansprüche

1. System zur Übertragung von Daten, einen Remote-Monitoring-Server, RMS, (1) und mindestens ein Remote-Gerät der Gruppe, die mindestens ein Patienten-Remote-Gerät, PR, (5) und mindestens ein Remote-Gerät, CP, für Gesundheitsfachkräfte, HCP, (3) enthält, umfassend, wobei jedes von dem mindestens einen Remote-Gerät und dem RMS ein Kommunikationsmodul (16, 17, 22) umfasst, das dafür konfiguriert ist, eine Kommunikationsverbindung zwischen dem RMS und dem jeweiligen mindestens einen Remote-Gerät für eine Datenübertragung herzustellen und zu halten, wobei jeder Kommunikationspfad ein unterschiedliches Kommunikationsprotokoll nutzt und/oder einen unterschiedlichen Service Set Identifier hat, wobei das Kommunikationsmodul dafür konfiguriert ist, Werte von mindestens einem einer Vielzahl von Konnektivitätsparametern von jedem der mindestens zwei Kommunikationspfade zwischen dem jeweiligen Remote-Gerät und dem RMS zu bestimmen und zu analysieren, und automatisch einen Kommunikationspfad von den mindestens zwei Kommunikationspfaden auszuwählen, um die Kommunikationsverbindung zwischen dem jeweiligen Remote-Gerät und dem RMS auf Grundlage der analysierten Werte des mindestens einen Konnektivitätsparameters herzustellen und/oder zu halten, wobei das Kommunikationsmodul dafür konfiguriert ist, den einen Kommunikationspfad auf Grundlage der aktuell bestimmten Werte des mindestens einen Konnektivitätsparameters und auf Grundlage der in der Vergangenheit bestimmten Werte des mindestens einen Konnektivitätsparameters auszuwählen.

2. System nach Anspruch 1, wobei die Vielzahl von Konnektivitätsparametern mindestens einen Parameter der Gruppe umfassen, die Netzwerkgeschwindigkeit, Ping-Zeit, Paketverlustrate in Prozent, Übertragungsrate, Signalstärke, Erfolgsrate der Kommunikation enthält.

3. System nach einem der vorstehenden Ansprüche, wobei das Kommunikationsmodul dafür konfiguriert ist, den einen Kommunikationspfad auf Grundlage der Analyse der Werte des mindestens einen Konnektivitätsparameters auszuwählen, der nur durch dieses selbst erkannt wurde.

4. System nach einem der Ansprüche 1 bis 2, wobei das Kommunikationsmodul dafür konfiguriert ist, den einen Kommunikationspfad auf Grundlage der Analyse der Werte des mindestens einen Konnektivitätsparameters auszuwählen, der durch dieses selbst erkannt wurde, und zusätzlich auf Grundlage der Analyse der Werte des mindestens einen Konnektivitätsparameters des Kommunikationsmoduls aller ähnlichen Remote-Geräte des Systems.

5. System nach einem der vorstehenden Ansprüche, wobei das Kommunikationsmodul dafür konfiguriert ist, Werte des mindestens einen Konnektivitätsparameters, die während einer Kommunikationssitzung bestimmt wurden, am Ende der jeweiligen Kommunikationssitzung in einem Datenspeicher des jeweiligen mindestens einen Remote-Geräts und/oder in einem Datenspeicher des RMS (1) zu speichern.

6. System nach einem der vorstehenden Ansprüche, wobei das Kommunikationsmodul dafür konfiguriert ist, mindestens einen KI-Algorithmus zur Analyse der bestimmten Werte des mindestens einen Konnektivitätsparameters zu verwenden.

7. System nach einem der vorstehenden Ansprüche, wobei das Kommunikationsmodul dafür konfiguriert ist, die bestimmten Werte des mindestens einen Konnektivitätsparameters zusammen mit dem Ort und/oder der Zeit der Bestimmung durch das jeweilige mindestens eine Remote-Gerät und/oder den RMS (1) zu speichern, zum Beispiel einen GNSS-Wert des Standortes.

8. Verfahren zur Übertragung von Daten in einem System, einen Remote-Monitoring-Server, RMS, (1) und mindestens ein Remote-Gerät der Gruppe, die mindestens ein Patienten-Remote-Gerät, PR, (5) und mindestens ein Remote-Gerät, CP, für Gesundheitsfachkräfte, HCP, (3) enthält, umfassend, wobei jedes von dem mindestens einen Remote-Gerät und dem RMS ein Kommunikationsmodul (16, 17, 22) umfasst, das dafür konfiguriert ist, eine Kommunikationsverbindung zwischen dem RMS und dem jeweiligen mindestens einen Remote-Gerät für eine Datenübertragung herzustellen und zu halten, wobei jeder Kommunikationspfad ein unterschiedliches Kommunikationsprotokoll nutzt und/oder einen unterschiedlichen Service Set Identifier hat, wobei das Kommunikationsmodul Werte von mindestens einem einer Vielzahl von Konnektivitätsparametern von jedem der mindestens zwei Kommunikationspfade zwischen dem jeweiligen Remote-Gerät und dem RMS bestimmt und analysiert, und automatisch einen Kommunikationspfad von den mindestens zwei Kommunikationspfaden auswählt, um die Kommunikationsverbindung zwischen dem jeweiligen Remote-Gerät und dem RMS auf Grundlage der analysierten Werte des mindestens einen Konnektivitätsparameters herzustellen und/oder zu halten, wobei die Vielzahl von Konnektivitätsparametern zum Beispiel mindestens einen Parameter der Gruppe umfassen, die Netzwerkgeschwindigkeit, Ping-Zeit, Paketverlustrate in Prozent, Übertragungsrate, Signalstärke, Erfolgsrate der Kommunikation enthält, wobei das Kommunikationsmodul den einen Kommunikationspfad auf Grundlage der aktuell bestimmten Werte des mindestens einen Konnektivitätsparameters und auf Grundlage der in der Vergangenheit bestimmten Werte des mindestens einen Konnektivitätsparameters auswählt.

9. Verfahren nach Anspruch 8, wobei das Kommunikationsmodul den einen Kommunikationspfad auf Grundlage der Analyse der Werte des mindestens einen Konnektivitätsparameters auswählt, der nur durch dieses selbst erkannt wurde.

10. Verfahren nach Anspruch 8, wobei das Kommunikationsmodul den einen Kommunikationspfad auf Grundlage der Analyse der Werte des mindestens einen Konnektivitätsparameters auswählt, der durch dieses selbst erkannt wurde, und zusätzlich auf Grundlage der Analyse der Werte des mindestens einen Konnektivitätsparameters des Kommunikationsmoduls aller ähnlichen Remote-Geräte des Systems.

11. Verfahren nach einem der Ansprüche 8 bis 10, wobei das Kommunikationsmodul Werte des mindestens einen Konnektivitätsparameters, die während einer Kommunikationssitzung bestimmt wurden, am Ende der jeweiligen Kommunikationssitzung in einem Datenspeicher des jeweiligen mindestens einen Remote-Geräts und/oder in einem Datenspeicher des RMS (1) speichert, und/oder wobei das Kommunikationsmodul mindestens einen KI-Algorithmus zur Analyse der bestimmten Werte des mindestens einen Konnektivitätsparameters verwendet, und/oder
wobei die bestimmten Werte des mindestens einen Konnektivitätsparameters durch das Kommunikationsmodul zusammen mit ihrem Ort und/oder ihrer Zeit der Bestimmung durch das jeweilige mindestens eine Remote-Gerät und/oder den RMS gespeichert werden, zum Beispiel ein GNSS-Wert des Standortes.

12. Computerprogrammprodukt, das Anweisungen umfasst, die bei Ausführung durch eine Datenverarbeitungseinheit die Datenverarbeitungseinheit veranlassen, die Schritte des Verfahrens nach einem der Ansprüche 8 bis 11 durchzuführen.

13. Computerlesbarer Datenträger, auf dem ein Computerprogrammprodukt nach Anspruch 12 gespeichert ist.

## Revendications

1. Système de transmission de données comprenant un serveur de surveillance à distance ou RMS (1), et au moins un dispositif distant du groupe comprenant au moins un dispositif distant de patient ou PR (5), et au moins un dispositif distant ou CP (3) de professionnel de santé ou HCP
dans lequel chacun parmi l'au moins un dispositif distant et le RMS comprend un module de communication (16, 17, 22) qui est configuré pour établir et maintenir une connexion en communication entre le RMS et l'au moins un dispositif distant respectif à travers un parmi au moins deux trajets de communication pour la transmission de données, dans lequel chaque trajet de communication utilise un protocole de communication différent et/ou a un identifiant d'ensemble de services différent, dans lequel le module de communication est configuré pour déterminer et analyser des valeurs d'au moins un parmi une pluralité de paramètres de connectivité de chacun des au moins deux trajets de communication entre le dispositif distant respectif et le RMS et pour choisir automatiquement un trajet de communication parmi les au moins deux trajets de communication afin d'établir et/ou de maintenir la connexion en communication entre le dispositif distant respectif et le RMS en se basant sur les valeurs analysées de l'au moins un paramètre de connectivité, dans lequel le module de communication est configuré pour choisir le trajet de communication en se basant sur les valeurs réellement déterminées de l'au moins un paramètre de connectivité et en se basant sur les valeurs de l'au moins un paramètre de connectivité déterminé par le passé.

2. Système selon la revendication 1, dans lequel la pluralité de paramètres de connectivité comprennent au moins un paramètre du groupe contenant la vitesse de réseau, le temps de latence, le pourcentage de perte de paquets, le taux de transfert, la force de signal, le taux de réussite de communication.

3. Système selon l'une quelconque des revendications précédentes, dans lequel le module de communication est configuré pour choisir le trajet de communication en se basant sur l'analyse des valeurs de l'au moins paramètre de connectivité détecté par lui-même uniquement.

4. Système selon l'une quelconque des revendications 1 à 2, dans lequel le module de communication est configuré pour choisir le trajet de communication en se basant sur l'analyse des valeurs de l'au moins un paramètre de connectivité détecté par lui-même et de plus en se basant sur l'analyse des valeurs de l'au moins un paramètre de connectivité du module de communication de tous les dispositifs distants similaires du système.

5. Système selon l'une quelconque des revendications précédentes, dans lequel le module de communication est configuré pour stocker des valeurs de l'au moins un paramètre de connectivité déterminé pendant une session de communication dans une mémoire de données de l'au moins un dispositif distant respectif et/ou dans une mémoire de données du RMS (1) au niveau de la fin de la session de communication respective.

6. Système selon l'une quelconque des revendications précédentes, dans lequel le module de communication est configuré pour utiliser au moins un algorithme d'IA pour l'analyse des valeurs déterminées de l'au moins un paramètre de connectivité.

7. Système selon l'une quelconque des revendications précédentes, dans lequel le module de communication est configuré pour stocker les valeurs déterminées de l'au moins un paramètre de connectivité conjointement avec la localisation et/ou le temps de détermination par l'au moins un dispositif distant respectif et/ou le RMS (1), par exemple une valeur de GNSS de la localisation.

8. Procédé de transmission de données dans un système comprenant un serveur de surveillance à distance ou RMS (1), et au moins un dispositif distant du groupe comprenant au moins un dispositif distant de patient ou PR (5), et au moins un dispositif distant ou CP (3) de professionnel de santé ou HCP,
dans lequel chacun parmi l'au moins un dispositif distant et le RMS comprend un module de communication (16, 17, 22) qui établit et maintient une connexion en communication entre le RMS et l'au moins un dispositif distant respectif à travers un parmi au moins deux trajets de communication pour la transmission de données, dans lequel chaque trajet de communication utilise un protocole de communication différent et/ou a un identifiant d'ensemble de services différent, dans lequel le module de communication détermine et analyse des valeurs d'au moins un parmi une pluralité de paramètres de connectivité de chacun des au moins deux trajets de communication entre le dispositif distant respectif et le RMS et choisit automatiquement un trajet de communication parmi les au moins deux trajets de communication afin d'établir et/ou de maintenir la connexion en communication entre le dispositif distant respectif et le RMS en se basant sur les valeurs analysées de l'au moins un paramètre de connectivité, dans lequel la pluralité de paramètres de connectivité comprennent, par exemple, au moins un paramètre du groupe contenant la vitesse de réseau, le temps de latence, le pourcentage de perte de paquets, le taux de transfert, la force de signal et le taux de réussite de communication, dans lequel le module de communication choisit le trajet de communication en se basant sur des valeurs réellement déterminées de l'au moins un paramètre de connectivité et en se basant sur les valeurs de l'au moins un paramètre de connectivité déterminé par le passé.

9. Procédé selon la revendication 8, dans lequel le module de communication choisit le trajet de communication en se basant sur l'analyse des valeurs de l'au moins paramètre de connectivité détecté par lui-même uniquement.

10. Procédé selon la revendication 8, dans lequel le module de communication choisit le trajet de communication en se basant sur l'analyse des valeurs de l'au moins un paramètre de connectivité détectée par lui-même et de plus en se basant sur l'analyse des valeurs de l'au moins un paramètre de connectivité du module de communication de tous les dispositifs distants similaires du système.

11. Procédé selon l'une quelconque des revendications 8 à 10, dans lequel le module de communication stocke des valeurs de l'au moins un paramètre de connectivité déterminé pendant une session de communication dans une mémoire de données de l'au moins un dispositif distant respectif et/ou dans une mémoire de données du RMS (1) au niveau de la fin de la session de communication respective et/ou
dans lequel le module de communication utilise au moins un algorithme d'IA pour l'analyse des valeurs déterminées de l'au moins un paramètre de connectivité et/ou
dans lequel les valeurs déterminées de l'au moins paramètrent de connectivité sont stockées par le module de communication conjointement avec leur localisation et/ou le temps de détermination par l'au moins un dispositif distant respectif et/ou le RMS, par exemple la valeur de GNSS de la localisation.

12. Produit de programme informatique comprenant des instructions qui, lorsqu'elles sont exécutées par une unité de traitement, amènent l'unité de traitement à réaliser les étapes du procédé selon l'une quelconque des revendications 8 à 11.

13. Support de données lisible par ordinateur stockant un produit de programme informatique selon la revendication 12.
